# EUROPEAN PATENT APPLICATION

(11) **EP 4 782 025 A1**
(43) Date of publication of application: **29.07.2026**
(21) Application number: 25154057.1
(22) Date of filing: 27.01.2025
(51) Int. Cl.: A61M 1/36, G01L 19/00

(54) **TRANSDUCER PROTECTOR FOR AN EXTRACORPOREAL CIRCUIT**

(71) Applicant: Fresenius Medical Care Deutschland GmbH, 61352 Bad Homburg (DE)
(72) Inventor: BECKER, Martin, 61352 Bad Homburg, (DE); CAVALLI, Riccardo, 61352 Bad Homburg, (DE)
(74) Representative: Lorenz Seidler Gossel Part. mbB

(57) **Abstract**

The present invention provides a first and a second a system for connecting a pipe of an extracorporeal circuit to a pressure transducer interface, the system comprising a transducer protector and an adapter, the transducer protector comprising a first main body comprising a first fluid path, a first connector for fluidly coupling a first end of the first fluid path to the pipe of the extracorporeal circuit, a second connector for releasably coupling a second end of the first fluid path to the adapter, and a hydrophobic gas-permeable membrane arranged in the first fluid path for separating the first end of the first fluid path from the second end; the adapter comprising a second main body comprising a second fluid path, a third connector for releasably coupling a first end of the second fluid path to the first fluid path of the transducer protector, and a fourth connector for releasably coupling a second end of the second fluid path to the pressure transducer interface. The second connector is a snap-fit connector and the third connector comprises a snap-fit counter-element for the second connector, or the fourth connector is a snap-fit connector configured to be connected to a counter-element of the pressure transducer interface.

## Description

The present invention relates to a transducer protector for protecting a pressure transducer connected to an extracorporeal blood circuit.

In medical treatments which require an extracorporeal circuit, in particular in blood-treatments such as hemodialysis, the pressure in the extracorporeal circuit, for example the arterial and/or the venous pressure, must be constantly monitored. This is usually achieved by means of a pressure transducer connected to the main circuit of the extracorporeal circuit by a branch pipe.

In order to protect the pressure transducer, which is typically located inside a dialysis machine, from any contact with the patient's blood, a transducer protector is usually provided between the pipe and the pressure transducer. In particular, the transducer protector may be provided as a part of the extracorporeal circuit as a disposable, and during treatment being connected to a pressure transducer interface.

Document EP 2 226 087 A1 shows a well-established configuration of a transducer protector for an extracorporeal circuit, comprising a main body comprising a fluid path, a first connector for fluidly coupling a first end of the fluid path to a pipe of the extracorporeal circuit, a second connector for fluidly coupling a second end of the fluid path to a pressure transducer interface, and a hydrophobic gas-permeable membrane arranged in the fluid path for separating the first end of the fluid path from the second end.

In this known transducer protector, the second connector is configured as a female type Luer-type connector, comprising a screw section for screwing the second connector onto a corresponding screw section of the pressure transducer interface. Further, the main body of the transducer protector is formed by means of two plastic half-shells enclosing between them a hydrophobic membrane, the two sections forming a circular section arranged between the two tubular connectors. The circular section enclosing the membrane is used as a gripping section for turning the transducer protector for closing and opening the Luer connection.

This transducer protector provides a robust design used for many years in the market. However, it has non-optimal usability and is in particular difficult to handle due to the reduced surface of the circular central element. Further, due to the rotating movement needed to screw the component on the Luer lock male connector, fixation is difficult. Finally, the user is not aware if the transducer protector is properly connected or not, and therefore not aware if the connection is tight. Further, manufacture of the transducer protector is difficult.

Despite these disadvantages, the standard configuration using two half shells and a Luer lock connection has nevertheless been the generally accepted solution for connecting a transducer protector to the corresponding pressure transducer interface in the market. So far, most efforts in improving known transducer protectors have centered on the improvement of the safety of the separation function of the membrane. For example, document EP 2 408 490 B1 shows a transducer protector using two membranes arranged in series.

Post-published Document EP 4 497 455 A1 of the same applicant shows a transducer protector having a snap-fit connector for connecting with a corresponding counter-element of the pressure transducer interface which is e.g. fixedly arranged on the dialysis machine.

This solution provides for an easier handling of the transducer protector. In particular, a snap-fit connector is decisively easier to handle than the Luer connectors used in the prior art. Further, the user is provided with a tactile and/or audible feedback, such that proper connection is ensured.

However, this solution requires a pressure transducer interface having a snap-fit counter element. Therefore, it cannot be used on older dialysis machines which still have an interface of a different type. Further, the pressure transducer interface is not easy to clean and might require a regular replacement of a sealing element.

Further, if the pressure transducer interface, which is e.g. fixedly arranged on the dialysis machine, only has a snap-fit counter element, standard pressure transducers having a different connector type cannot be used on the new machines.

The object of the present invention is to provide a system to avoid at least one of these disadvantages.

This object is solved in two independent aspects by a system according to claims 1 and 7 as well as a corresponding adapter according to claim 12.

In a first independent aspect, the present invention comprises a system for connecting a pipe of an extracorporeal circuit to a pressure transducer interface, the system comprising a transducer protector and an adapter, the transducer protector comprising a first main body comprising a first fluid path, a first connector for fluidly coupling a first end of the first fluid path to the pipe of the extracorporeal circuit, a second connector for releasably coupling a second end of the first fluid path to the adapter, and a hydrophobic gas-permeable membrane arranged in the first fluid path for separating the first end of the first fluid path from the second end; the adapter comprising a second main body comprising a second fluid path, a third connector for releasably coupling a first end of the second fluid path to the first fluid path of the transducer protector, and a fourth connector for releasably coupling a second end of the second fluid path to the pressure transducer interface; wherein the second connector is a snap-fit connector and the third connector comprises a snap-fit counter-element for the second connector.

Therefore, the adapter provides a snap-fit counter element to which a transducer protector can be snap-fit connected.

Therefore, the adapter can be used to equip a device having a different type of connector as a transducer protector interface with a snap-fit counter element. Therefore, older machines can be used with the more advantageous snap-fit transducer protectors such as shown in EP 4 497 455 A1.

Further, the adapter can be easily replaced by a new adapter. Thereby, the hygienic situation is improved, because a fresh adapter can be provided at regular intervals. Further, the sealing properties of the snap-fit connection can equally be maintained by a regular replacement of the adapter.

In an embodiment of the present invention, the second and third connector are configured to be snap-fit engaged with a single axial movement of the transducer protector in a direction defined by the second fluid path at is first end. Thereby, handling of the connection between the transducer protector and the adapter is simplified.

In an embodiment of the present invention, the first end of the second fluid path of the adapter is configured as a third tubular element to be sealingly engaged with the second end of the first fluid path of the transducer protector.

In an embodiment of the present invention, the adapter comprises a sealing element to sealingly engage with the second end of the first fluid path of the transducer protector and in particular with a second tubular element of the transducer protector forming the second end of the first fluid path of the transducer protector.

If such a sealing element has to be replaced at regular intervals, with the adapter, it is no longer necessary to remove the sealing element from the sealing geometry, which might need a service technician or might lead to damages on the sealing geometry if not properly done, because the entire adapter can be replaced in order to provide a new sealing element.

In an embodiment of the present invention, the sealing element is provided on an outer surface of the third tubular element to engage with the inner surface of a second tubular element of the second connector.

In an embodiment of the present invention, the sealing element is a sealing ring arranged in a groove provided on the outer surface of the third tubular element.

In an embodiment of the present invention, the third tubular element has an outer surface to engage with an inner surface of the second tubular element, wherein preferably, the outer surface is cone-shaped. The tubular element as well as the cone shape will help to guide the second tubular element on the third tubular element in order to avoid a misalingement.

In an embodiment of the present invention, in an axial direction, a counter-geometry of the counter-element is provided between a first axial end and a second axial end of the outer surface of the third tubular element. This provides a particular advantageous force distribution and a relatively short product.

In an embodiment of the present invention, in a radial direction, the counter-element is provided on an outer side of the third tubular element with a free space provided between the counter-element and the third tubular element to accommodate the second tubular element and/or a snap-fit connection element of the second connector.

In an embodiment of the present invention, the counter-element has at least one snap-fit counter-geometry to snap-fit engage with a connection element of the second connector.

In an embodiment of the present invention, the snap-fit counter-geometry is arranged on an inner side of the counter-element to engage with a snap-fit geometry arranged on an outer side of the connecting element.

In an embodiment of the present invention, the snap-fit counter-geometry comprises a hook section for snap engaging with a hook section of the snap-fit geometry of the second connector and preferably a chamfer section.

In an embodiment of the present invention, the hook section and/or the chamfer section extend circumferentially in an arc section around an axis defined by the second fluid path at its first end.

In an embodiment of the present invention, the third connector comprises at least two counter-elements to engage with corresponding connection elements of the second connector.

In an embodiment of the present invention, the counter-elements are arranged on opposite sides of an axis defined by the second fluid path at its first end.

In an embodiment of the present invention, the at least two counter-elements are arranged on a wall extending circumferentially around the first end of the second fluid path.

In an embodiment of the present invention, the wall extends at a distance to a third tubular element of the third connector, the third tubular element forming the second fluid path at its first end.

In an embodiment of the present invention, the at least two counter-elements extend from a free end of the wall to an inside towards the second fluid path.

In an embodiment of the present invention, the wall may be connected to the third tubular element of the adapter by a bottom wall.

In an embodiment of the present invention, the wall and/or the bottom wall are formed as closed surfaces. Alternatively, the wall and/or the bottom wall may have openings and/or may be provided by web elements.

In an embodiment of the present invention, the wall an the bottom wall together have a cup shape, with the third tubular element preferably extending from the bottom wall towards the open side of the cup shape.

In an embodiment of the present invention, the fourth connector is configured to sealingly engage a pressure transducer interface.

The fourth connector may be any type of connector, and in particular may be a standard connector as used in the art.

In an embodiment of the present invention, the fourth connector is of a different connector type than the third connector. In the context of the present invention, the feature that the fourth connector is of a different connector type than the third connector is equivalent to saying that the fourth connector could not be connected to the third connector and/or vice versa.

The adapter thereby allows to engage the transducer protector having a snap-fit connector to a pressure transducer interface having a connector type to which the snap-fit connector of the transducer protector could not be connected.

In an embodiment of the present invention, the fourth connector is a Luer connector.

In an embodiment of the present invention, the fourth connector has a sealing cone and a screw element to engage with corresponding geometries of the pressure transducer interface.

In an embodiment of the present invention, the fourth connector is configured as a female Luer connector. Thereby, the fourth connector is configured to be connected to a male Luer connector provided as a pressure transducer interface on many machines on the market.

In an embodiment of the present invention, the fourth connector comprises a fourth tubular element forming the second end of the second fluid path.

In an embodiment of the present invention, the fourth tubular element has an inner surface configured as a sealing cone and a screw element arranged on its outer surface.

In an embodiment of the present invention, the second fluid path extends along a single direction through the adapter.

In an embodiment of the present invention, the adapter is integrally formed as a single element, in particular by injection molding.

As a further independent aspect, the present invention further comprises the adapter of the system as described above.

The adapter will usually be provided separately from the transducer protector, because the transducer protector will usually be part of a disposable to be replaced after each use or at least after a plurality of uses, while the adapter may stay on the machine for a longer time and therefore be used for connecting a plurality of transducer protectors to the pressure transducer interface.

The adapter may be configured as described above.

In particular, the adapter may comprise a second main body comprising a second fluid path, a third connector for releasably coupling a first end of the second fluid path to the first fluid path of the transducer protector, and a fourth connector for releasably coupling a second end of the second fluid path to the pressure transducer interface, wherein the third connector is a snap-fit counter-element configured for connecting a snap-fit connector of the transducer protector.

For further configurations of the adapter, we refer to the above description of the system.

The present invention further comprises a blood treatment machine, in particular a dialysis machine, comprising a pressure transducer, a pressure transducer interface and an adapter and/or a system as described above, wherein the adapter and the pressure transducer interface are configured to be releasably connected to each other.

The present invention further comprises the use of the system and/or the adapter described above for connecting a pipe of an extracorporeal circuit to a pressure transducer.

In an embodiment, the transducer protector is discarded after each use or a plurality of uses and the adapter remains arranged on the pressure transducer interface to be used consecutively with a plurality of transducer protectors.

According to embodiments of the present invention, the transducer protector may be configured as described in EP 4 497 455 A1, the content of which is hereby included by reference in its entirety.

Further, the third connector of the adapter may be configured in the same way as the pressure transducer interface described in EP 4 497 455 A1, the content of which is hereby included by reference in its entirety.

In a second independent aspect, the present invention comprises a system for connecting a pipe of an extracorporeal circuit to a pressure transducer interface, the system comprising a transducer protector and an adapter, the transducer protector comprising a first main body comprising a first fluid path, a first connector for fluidly coupling a first end of the first fluid path to the pipe of the extracorporeal circuit, a second connector for releasably coupling a second end of the first fluid path to the adapter, and a hydrophobic gas-permeable membrane arranged in the first fluid path for separating the first end of the first fluid path from the second end; the adapter comprising a second main body comprising a second fluid path, a third connector for releasably coupling a first end of the second fluid path to the first fluid path of the transducer protector, and a fourth connector for releasably coupling a second end of the second fluid path to the pressure transducer interface; wherein the fourth connector is a snap-fit connector configured to be connected to a counter-element of the pressure transducer interface.

The second aspect and in particular the adapter according to the second aspect can be used to equip a device having a snap-fit counter element as a connecter of the pressure transducer interface with a different counter element provided by the third connector to connect other types of transducer protectors to the device. Therefore, new machines equipped with a snap-fit counter element, such as shown e.g. in EP 4 497 455 A1, can still be used with other transducer protectors, such as standard Luer type transducer protectors.

In other words, the system according to the second aspect, i.e. the adapter according to the second aspect together with the corresponding transducer protector will provide a two-part transducer protector having a snap-fit connector as defined in EP 4 497 455 A1.

Further details of the second aspect will be described in the following:
In an embodiment of the present invention, the fourth connector is configured to be snap-fit engaged with a single axial movement of the adapter in a direction defined by the second fluid path at is second end.

In an embodiment of the present invention, the fourth connector comprises at least one connection element connected to a main body of the adapter and having a free end comprising a connection geometry for snap engaging the counter element of the pressure transducer interface.

In an embodiment of the present invention, the connection element extends from a connection point where it is connected to the main body of the adapter in a direction substantially along of the second fluid path of the adapter towards the pressure transducer interface.

In an embodiment of the present invention, the second end of the second fluid path is configured as a fourth tubular element to be sealingly engaged with a fluid path of the pressure transducer interface.

In an embodiment of the present invention, the at least one connection element of the fourth connector extends from a connection point with the main body of the adapter towards the pressure transducer interface on an outside and further preferably with a distance to the fourth tubular element.

In an embodiment of the present invention, the connection geometry of the at least one connection element of the fourth connector is arranged on an outer side of the connection element with respect to a radial direction.

In an embodiment of the present invention, the connection geometry comprises a chamfer section and a hook section.

In an embodiment of the present invention, the fourth connector comprises at least two separate connection elements, the connection elements being preferably arranged on opposite sides of an axis defined by the second fluid path at its second end.

In an embodiment of the present invention, the fourth connector comprises at least one snap-fit element of the cantilever type.

In an embodiment of the present invention, the snap-fit element is a snap-fit arm connected to a main body of the adapter and having a free end comprising a snap-fit geometry for snap engaging the counter element of the pressure transducer interface.

In an embodiment of the present invention, the snap-fit arm extends from a connection point where it is connected to the main body of the adapter in a direction substantially along of the second fluid path of the adapter.

In an embodiment of the present invention, the snap-fit connection provided by the fourth connector is a releasable connection.

In an embodiment of the present invention, the fourth connector is configured such that the snap-fit connection is releasable by flexing at least one snap-fit element by applying pressure to a handling section of the snap-fit element.

In an embodiment of the present invention, the snap-fit element is configured to be releasable by a pressure acting on the handling section of the snap-fit element from an outer side in a direction towards the main body of the connector.

In an embodiment of the present invention, the fourth connector comprises at least two separate snap-fit elements.

In an embodiment of the present invention, the snap-fit elements are arranged on opposite sides of an axis defined by the second fluid path at its second end.

In an embodiment of the present invention, the snap-fit connection provided by the second connector is releasable by pressing the handling sections of the snap-fit elements from an outer side in a direction towards each other.

In an embodiment of the present invention, a snap-fit geometry of the fourth connector is configured to snap engage a counter element of the pressure transducer interface from an inner side.

In an embodiment of the present invention, a handling section of at least one snap-fit element of the adapter is arranged between a connection point where the snap-fit element is connected to the main body of the adapter and a free end of the snap-fit element configured for connecting to the pressure transducer interface.

In an embodiment of the present invention, the third connector is configured to sealingly engage with the second connector of the transducer protector.

In an embodiment of the present invention, the third connector is of a different connector type than the fourth connector.

In the context of the present invention, the feature that the third connector is of a different connector type than the fourth connector is equivalent to saying that the fourth connector could not be connected to the third connector and/or vice versa.

In an embodiment of the present invention, the third connector has a sealing cone and a screw element to engage with corresponding geometries of the second connector.

In an embodiment of the present invention, the third connector is configured as a Luer connector, in particular a male Luer cone connector.

In an embodiment of the present invention, the third connector comprises a male sealing cone forming the first end of the second fluid path and a third tubular element extending around the male sealing cone and comprising a screw element arranged on its inner surface.

In an embodiment of the present invention, the second fluid path extends along a single direction through the adapter.

In an embodiment of the present invention, the adapter is integrally formed as a single element, in particular by injection molding.

As a further independent aspect, the present invention further comprises the adapter of the system according to the second aspect as described above.

The adapter may be provided separately from the transducer protector, because the transducer protector will usually be part of a disposable to be replaced after each use or at least after a plurality of uses, while the adapter may stay on the machine for a longer time and therefore be used for connecting a plurality of transducer protectors to the pressure transducer interface. However, the adapter may also be provided together and/or discarded together with the transducer protector.

The adapter may be configured as described above.

In particular, the adapter may comprise a second main body comprising a second fluid path, a third connector for releasably coupling a first end of the second fluid path to the first fluid path of the transducer protector, and a fourth connector for releasably coupling a second end of the second fluid path to the pressure transducer interface, wherein the fourth connector is a snap-fit connector configured for connecting a snap-fit counter-element of the pressure transducer interface.

For further configurations of the adapter, we refer to the above description of the system according to the second aspect.

The present invention further comprises a blood treatment machine, in particular a dialysis machine, comprising a pressure transducer, a pressure transducer interface and an adapter and/or a system as described above with respect to the second aspect, wherein the adapter and the pressure transducer interface are configured to be releasably connected to each other.

The present invention further comprises an extracorporeal blood tubing set comprising a pipe and a system as described above for the second aspect, with the pipe being connected to the first connector, wherein preferably the pipe is permanently connected to the first connector.

The present invention further comprises the use of the system and/or the adapter described above for connecting a pipe of an extracorporeal circuit to a pressure transducer.

In an embodiment, the transducer protector is discarded after each use or a plurality of uses and the adapter remains arranged on the pressure transducer interface to be used consecutively with a plurality of transducer protectors.

In an embodiment, the adapter is provided together and/or discarded together with the transducer protector after each use or a plurality of uses

According to embodiments of the present invention, the fourth connector of the adapter according to the second aspect may be configured in the same way as the second connector of the transducer protector described in EP 4 497 455 A1, the content of which is hereby included by reference in its entirety.

Further, the pressure transducer interface according to the second aspect may be configured in the same way as the pressure transducer interface described in EP 4 497 455 A1, the content of which is hereby included by reference in its entirety.

Further details of the transducer protector, the pressure transducer interface, the snap-fit connector and the snap-fit counter element of both the second and the first aspect of the present invention will be described in the following:
As discussed above, a hydrophobic gas-permeable membrane is arranged in the first fluid path of the transducer protector for separating the first end of the first fluid path from the second end.

The membrane may comprise a polytetrafluoroethylene (ePTFE) polymer.

In one embodiment the membrane is made of a PFAS-free polymeric material. The PFAS-free polymeric material may be selected from polyurethane (PU), polyethylene (PE), polypropylene (PP), polyester, polyethylene terephthalate (PET), polycarbonate (PC), polyimide (PI), polyethylene naphthalate (PEN), polyethersulfone (PES), polyether ether ketone (PEEK), and combinations thereof.

The membrane may comprise one or more reinforcement layers and/or one or more coating layers.

In an embodiment of the present invention, the adapter comprises a hydrophobic gas-permeable membrane arranged in the second fluid path that separates the first end from the second end of the second fluid path.

Such an adapter could be used with a transducer protector as defined above, with the transducer protector not comprising a membrane.

Therefore, the present invention also comprises the systems as defined above with a transducer protector as defined above, wherein the transducer protector however does not comprise a membrane, but the adapter is provided with a membrane.

However, as the adapter is intended, at least according to an embodiment, to stay on the pressure transducer interface for a plurality of uses, the transducer protector preferably comprises a membrane in order to provide a barrier between the extracorporeal circuit and the adapter.

In an embodiment of the present invention, the adapter does not comprise a membrane arranged in the second fluid path that separates the first end from the second end of the second fluid path, i.e. the first and the second end of the fluid path are directly connected. Providing an additional membrane in the adapter would increase the resistance of the system with respect to pressure changes and therefore would reduce the sensitivity of the pressure transducer.

In an embodiment of the present invention, the first connector is a tubular element configured for receiving an end section of the pipe of the extracorporeal circuit.

In an embodiment of the present invention, the end section of the pipe is glued into the tubular element.

In an embodiment of the present invention, the first connector comprises a receiving section for a third connector. The third connector may, e.g., be a male or female Luer connector, a bayonet connector or screw-type connector.

In an embodiment of the present invention, the snap-fit geometry comprises a hook section for snap engaging a hook section of the counter element.

In an embodiment of the present invention, the snap-fit arm comprises a chamfer section arranged at its free end, the hook section arranged behind the chamfer section.

The chamfer section may be configured for contacting a front side of the counter element, such contact deforming the snap-fit arm when the snap-fit arm is moved in an axial direction until the hook section of the snap-arm engages with the hook section on the counter element.

In an embodiment of the present invention, the snap-fit arm extends from a connection point where it is connected to the main body in a direction along of the fluid path of the respective element. In particular, the snap-fit arm may extend with an angle of less than 20° with respect to an axial direction defined by the direction of the fluid path.

In a first variant, the snap-fit arm extends from the connection point only in the direction towards the counter element. Thereby, the handling area can be increased.

In a second variant, the snap-fit arm comprises a first section extending from the connection point in a direction away from the counter element and a second section extending from the connection point in a direction towards the counter element, each section having a fee end. The first section extending from the connection point in a direction away from the counter element may form a handling area for gripping the transducer protector.

In an embodiment of the present invention, the snap-fit connection is a releasable connection.

In an embodiment of the present invention, the snap-fit connection is releasable by flexing at least one snap-fit element by applying pressure to a handling section of the snap-fit element. This ensures that the snap-fit connection can be reliably disengaged.

In an embodiment of the present invention, the snap-fit element is configured to be releasable by a pressure acting on the handling section of the snap-fit element from an outer side in a direction towards the main body.

In an embodiment of the present invention, the snap-fit element is a snap-fit arm as defined herein, and in particular a snap-fit arm as described above.

In an embodiment of the present invention, there are at least two separate snap-fit elements. The snap-fit elements may each be configured as described above for the at least one snap-fit element.

In an embodiment of the present invention, the snap-fit elements are arranged on opposite sides of an axis defined by the fluid path.

In an embodiment of the present invention, the snap-fit connection is releasable by pressing the handling sections of the snap-fit elements from an outer side in a direction towards each other. This allows easy operation of the transducer protector.

In an embodiment of the present invention, the snap-fit elements each are a snap-fit arm as described herein.

In an embodiment of the present invention, the second end of the fluid path is configured as a tubular element, wherein a snap-fit element or snap-fit elements extend from a connection point with the main body towards the counter element on an outside and with a distance to the tubular element, the snap-fit element or snap-fit elements being deformable towards and/or away from the tubular element. This allows a reliable sealing provided by the tubular element combined with a reliable snap-fit connection provided by the snap-fit element or snap-fit elements without any interference of the one with the other.

In an embodiment of the present invention, a snap-fit geometry of the second connector is configured to snap engage a counter element from an inner side. In particular, the snap-fit geometry may therefore be provided on the snap-fit element or elements on an outer side.

In an embodiment of the present invention, a handling section of at least one snap-fit element of the second transducer is arranged between a connection point where the snap-fit element is connected to the main body and a free end of the snap-fit element. Thereby, a particularly large handling section can be provided.

In a second variant, a snap-fit geometry of the second connector is configured to snap engage a counter element from an outer side.

In an embodiment of the present invention, a handling section of at least one snap-fit element is arranged between a connection point where the snap-fit element is connected to the main body and a free end of a first section of the snap-fit element extending in a direction away from the counter element.

In an embodiment of the present invention, the snap-fit arm extends in a direction along of the fluid path towards the counter element, wherein the length of the snap-fit arm in a direction defined by the fluid path is at least 50% of the length of the fluid path, preferably at least 65%, further preferably at least 80%. This simplifies the handling of the snap connection.

In an embodiment of the present invention, a handling section of the snap-fit element is provided with ridges, the ridges preferably extending in a direction of the fluid path.

In an embodiment of the present invention, a snap-fit element or a snap-fit geometry has a rounded shape, preferably an essentially circular shape with respect to an axis defined by the fluid path. This allows for a particularly efficient connection. In particular, the shape may be circular.

In an embodiment of the present invention, in a cross section, an outer and/or an inner edge of the snap-fit element or a snap-fit geometry has a rounded shape, preferably an essentially circular shape. In particular, the shape may be circular.

In a first variant, the transducer protector is formed by two molded parts, each part comprising one of the first and the second connector, and/or wherein the hydrophobic gas-permeable membrane is arranged between the two molded parts.

In an embodiment, the first part comprises the first connector and the snap-fit arms.

In an embodiment, the second part comprises a second tubular element for connecting the fluid path to the adapter. Preferably, the inner diameter of the second tubular element is larger than a diameter of the membrane and/or comprises a bottom section protecting the membrane, the bottom section having a hole.

In an embodiment, the second tubular element extends from an outer edge of the bottom section towards the second side and in particular has, together with the bottom section, a pot shape.

In a second variant of the present invention, the transducer protector is formed by an integrally molded part comprising the entire fluid path and preferably both the first and the second connector. This reduces the production costs.

In a third variant of the present invention, which may be combined with the second variant, the hydrophobic gas-permeable membrane is bonded to a main body of the transducer protector from the side of the second end of the fluid path. In particular, the hydrophobic gas-permeable membrane is bonded to a step portion provided within the fluid path of the transducer protector facing the second end.

In an embodiment of the present invention, the integrally molded part comprises the entire fluid path and at least the sealing sections of the first and the second connector configured for sealingly connecting to the pipe and the adapter.

In an embodiment of the present invention, the integrally molded part comprises the entire fluid path, with the second end of the fluid path being formed by a tubular element configured for sealingly connecting to the adapter, and in particular to a sealing element of the adapter.

In a first variant, the snap-fit elements may be provided as a separate part connected to the integrally molded part.

In second, preferred variant the integrally molded part comprises the first and the second connector, i.e. all the parts of the first and the second connector.

In an embodiment of the present invention, the integrally molded part is the only structural part of the transducer protector.

In an embodiment of the present invention, the transducer protector is formed by the integrally molded part and the hydrophobic gas-permeable membrane, and optionally one or more sealing elements, with no further parts attached to transducer protector.

According to an aspect of the present invention, the hydrophobic gas-permeable membrane is bonded to a step portion of the transducer protector, the bonding area of the hydrophobic gas-permeable membrane being accessible from the second end of the fluid path.

In an embodiment, the step portion is provided within the fluid path or at the second end of the fluid path.

In an embodiment of the present invention, the inner diameter of the fluid path between its second end and the step portion is larger than the inner diameter of the fluid path at the step portion.

In an embodiment of the present invention, the inner diameter and/or the cross-section of the fluid path at its second end, and preferably between its second end and the hydrophobic gas-permeable membrane is larger than the outer diameter and/or outer shape of the hydrophobic gas-permeable membrane.

This simplifies the mounting of the hydrophobic gas-permeable membrane to the step portion, because the membrane and/or a bonding tool can be inserted into the transducer protector through the second end for manufacturing the transducer protector.

In an embodiment of the present invention, the second end of the fluid path is formed by a tubular element configured for sealingly connecting to the adapter, an inner diameter of the tubular element being larger than the inner diameter of a tubular element forming the first connector. Preferably, the inner diameter of the tubular element forming the second end of the fluid path is at least 120 % of the inner diameter of a tubular element forming the first connector, preferably at least 150 %. These features are preferred for transducer protectors according to all aspects of the present invention.

In an embodiment of the present invention, the inner diameter and/or the inner cross-section of the step portion is larger than the inner diameter and/or the cross-section of the first connector and/or the pipe connected thereto. This provides an effective surface of the hydrophobic gas-permeable membrane that is larger than the cross-section of the first connector and/or the pipe connected thereto.

In an embodiment of the present invention, the snap-fit counter element comprises a sealing element for sealingly engaging a tubular section of the snap-fit connector. The sealing element may contact a front surface of the tubular element and/or an inner or outer surface of the tubular element. The sealing element may be provided by a sealing ring or a conical section.

In an embodiment of the present invention, the sealing element engages with a front surface of the tubular section or a side surface of the tubular section.

In an embodiment of the present invention, the sealing element is formed by a cone section engaging with an inner cone section of the tubular section, in particular with a Luer cone section.

In an embodiment of the present invention, the snap-fit counter element is formed by a bracket having a middle section for engaging with the snap-fit geometry of the snap-fit element, the bracket freely extending between two connection sections where it is connected to a main surface of the main body. The bracket may in particular project from the main surface towards the element comprising the snap-fit connector.

The brackets allow a user to access from below the bracket to easily clean/disinfect around the fluid path.

In an embodiment of the present invention, the main surface is an essentially flat surface from which the bracket projects in a direction towards the transducer protector.

In a variant, the counter element is formed by an undercut of a recess provided in a main surface of the pressure transducer interface.

The counter element and/or the snap-fit connector may have a feature to prevent the improper mounting, in particular to avoid a mounting in a position that is rotated by 90° around its axis.

In an embodiment of the present invention, the pressure transducer interface comprises, in addition to the snap-fit counter element, a threaded section configured for engaging with another type of transducer protector comprising a Luer connector.

Such a pressure transducer interface is compatible both with prior art transducer protectors and with the inventive transducer protector.

The present invention further comprises a tubing system comprising a transducer protector as defined herein and at least one component of an extracorporeal blood circuit.

In particular, the component of the extracorporeal blood circuit is connected to the first connector via a tube, preferably glued to or inserted into a section of the first connector.

Preferably, the tube is a tube leading from a venous chamber of the extracorporeal blood circuit for conveying air across the hydrophobic gas-permeable membrane.

The component of the extracorporeal blood circuit may in particular be a venous chamber.

The tubing system may comprise further components of an extracorporeal blood circuit, such as a venous and/or an arterial line or connection lines connectable to a venous and/or an arterial line.

In an embodiment, the pressure transducer interface may be provided on a casing of the blood treatment machine. The main surface of the pressure transducer interface may be provided by an outer surface of the casing.

The blood treatment machine may further comprise an extracorporeal blood circuit.

In the context of the present invention, the terms axial and radial are always defined with respect to an axis provided by the respective fluid path and/or tubular element.

The present invention is now described in further detail with respect to drawings and embodiments.

In the drawings, the figures show the following:
- Fig. 1: a first embodiment of a transducer protector of the present invention in a side view,
- Fig. 2: the first embodiment in a perspective view when seen from the first side,
- Fig. 3: the first embodiment of a transducer protector, coupled to a first embodiment of a pressure transducer interface according to the present invention, in sectional view,
- Fig. 4: the first embodiment of a transducer protector connected to a second embodiment of a pressure transducer interface according to the present invention, in a sectional view,
- Fig. 5: the first embodiment of the transducer protector coupled to a third embodiment of a pressure transducer interface according to the present invention, in a sectional view,
- Fig. 6: a fourth embodiment of a pressure transducer interface in two perspective views,
- Fig. 7: the fourth embodiment of the pressure transducer interface with the first embodiment of the transducer protector connected thereto, in two perspective views,
- Fig. 8: a second embodiment of a transducer protector connected to a firth embodiment of a pressure transducer interface in a sectional view,
- Fig. 9: a third embodiment of a transducer protector coupled to a sixth embodiment of a pressure transducer interface in a sectional view,
- Fig. 10: a fourth embodiment of a transducer protector in a perspective view,
- Fig. 11: a fifth embodiment of a transducer protector in a perspective view,
- Fig. 12: a sixth embodiment of a transducer protector in a perspective view,
- Fig. 13: a seventh embodiment of a transducer protector in a cut view,
- Fig. 14: the seventh embodiment in a perspective view,
- Fig. 15: two variants of the seventh embodiment in a perspective view,
- Fig. 16: a fifth embodiment of a pressure transducer interface in a perspective view, and
- Fig. 17: an embodiment of a tubing set comprising a component of an extracorporeal blood circuit and the inventive transducer protector,
- Fig. 18: an embodiment of a system and an adapter according to the first aspect of the present invention in a cut view along an axis defined by the fluid path,
- Fig. 19: a perspective view of the adapter shown in Fig. 18,
- Fig. 20: a cut view of the adapter shown in Fig. 18 and 19,
- Fig. 21: an embodiment of an adapter according to the second aspect of the present invention in a cut view along an axis defined by the fluid path.

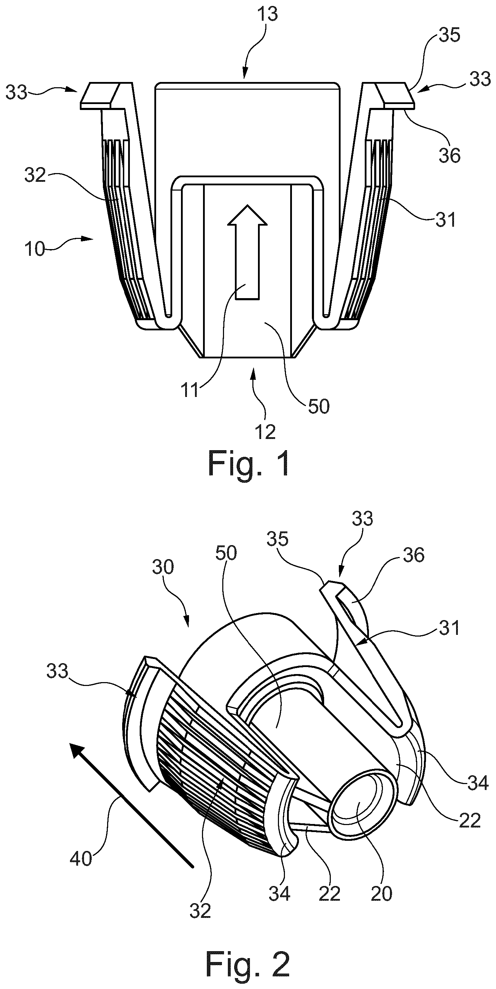

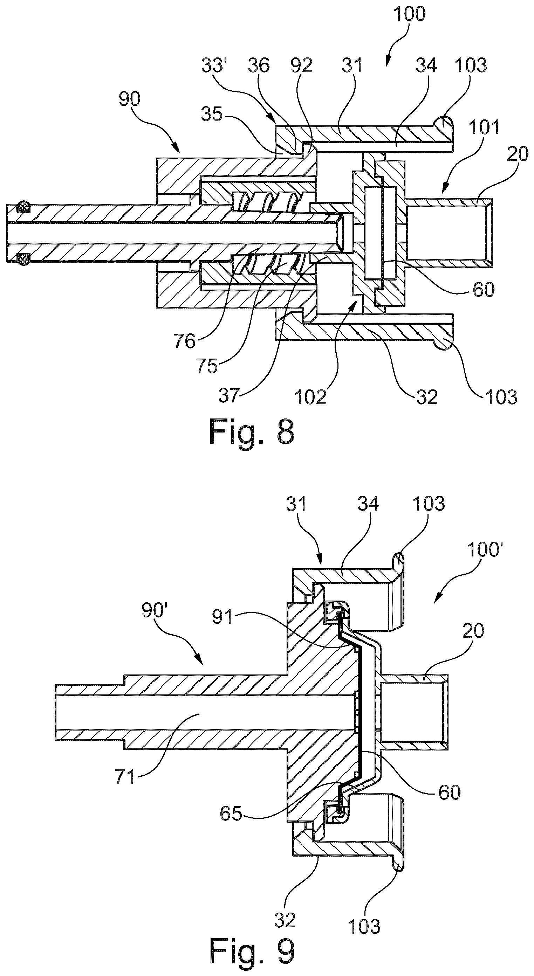

Fig. 1 - 5 and 7 show a first embodiment of a transducer protector according to the present invention. Further embodiments are shown in Fig. 8 to 15.

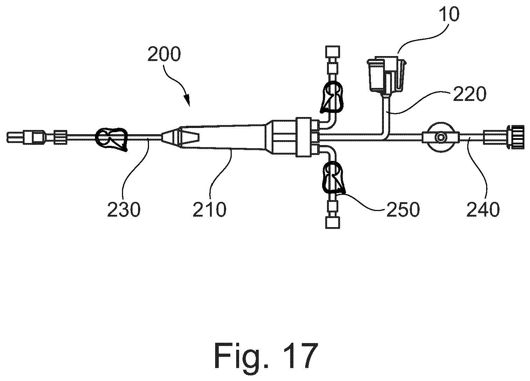

Fig. 17 shows an embodiment of a tubing set comprising an extracorporeal blood circuit and the inventive transducer protector. If not otherwise stated, the features and the configuration of the transducer protector described in the following applies to all the embodiments.

Further, the first embodiment of a transducer protector, as well as most of the other embodiments, embody all the aspects of the present invention in combination. However, the features described with respect to the respective aspects could also be used independently from the other aspects.

According to the present invention, the transducer protector 10 comprises a main body 50 comprising a (first) fluid path 11 schematically shown by an arrow in Fig. 1.

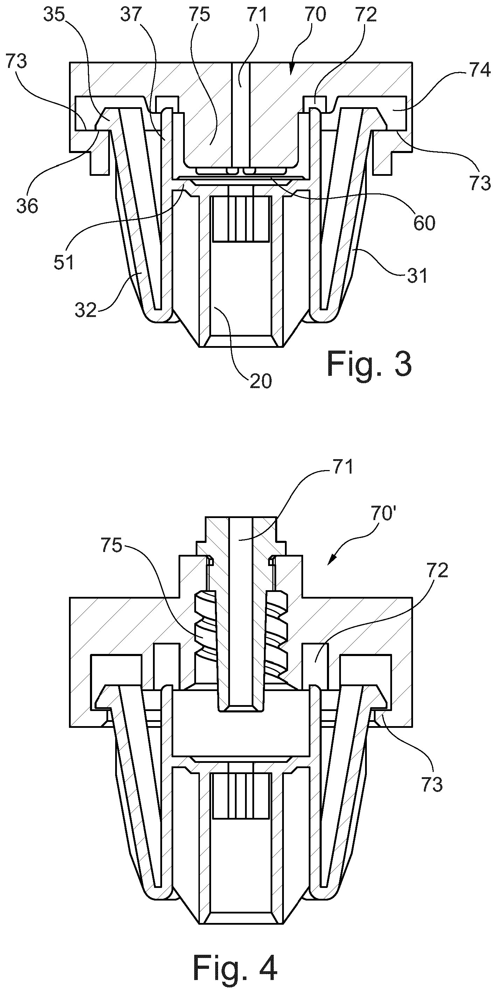

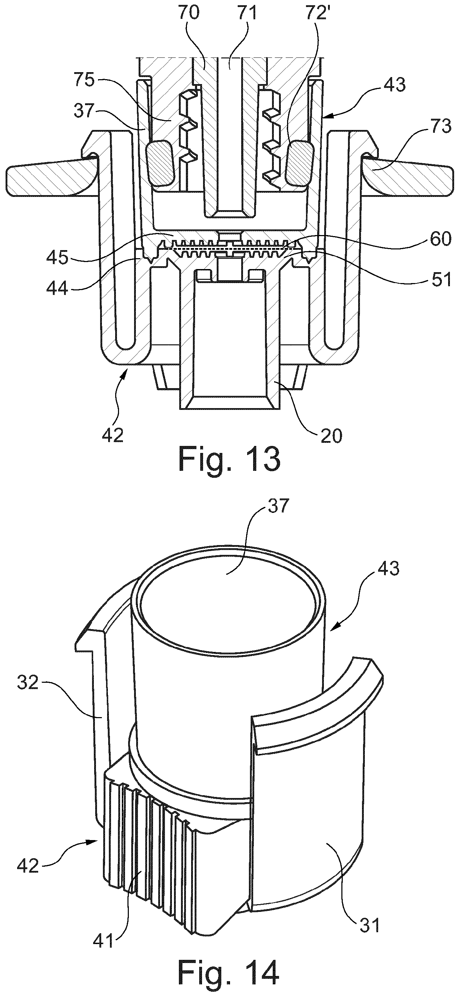

The fluid path 11 passes through the main body of the transducer protector from a first end 12 to a second end 13. Further, a hydrophobic gas-permeable membrane 60 shown in Fig. 3 and 13 is arranged in the fluid path 11 for separating the first end of the fluid path from the second end.

The hydrophobic gas-permeable membrane is in particular an air-permeable hydrophobic membrane. It therefore provides an air-permeable and therefore pressure-permeable connection through the connector, but is impermeable to liquids and therefore protects the second end 13 from blood entering through the first end 12.

The transducer protector 10 comprises a first connector 20 for fluidly coupling the first end 12 of the fluid path to a pipe of an extracorporeal circuit. In particular, as shown in Fig. 2 and 3, the first connector may be provided by a tubular element 20, into which the distal end of the pipe of the extracorporeal circuit may be inserted. The end of the pipe may be bonded to the first connector and in particular to the inside of the tubular element 20 by an adhesive.

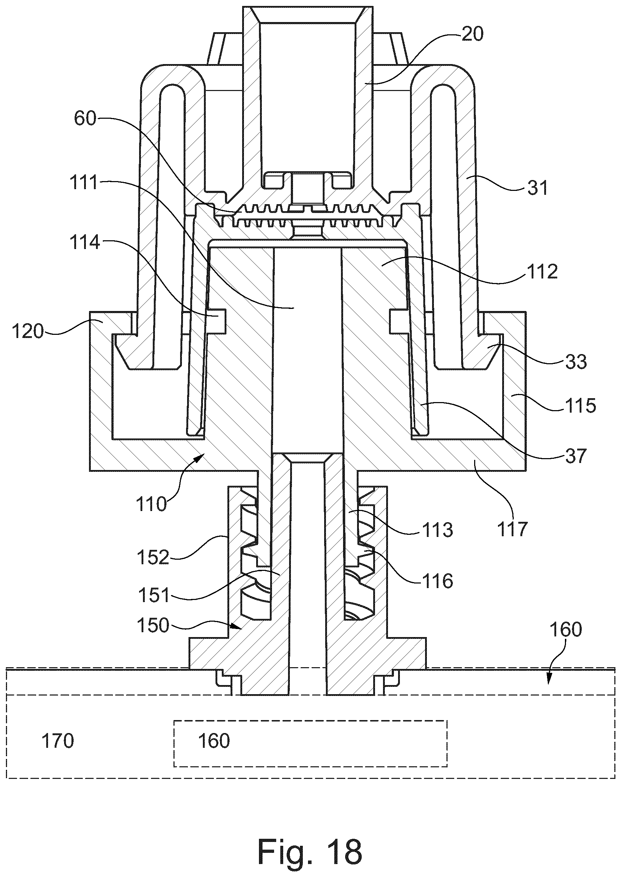

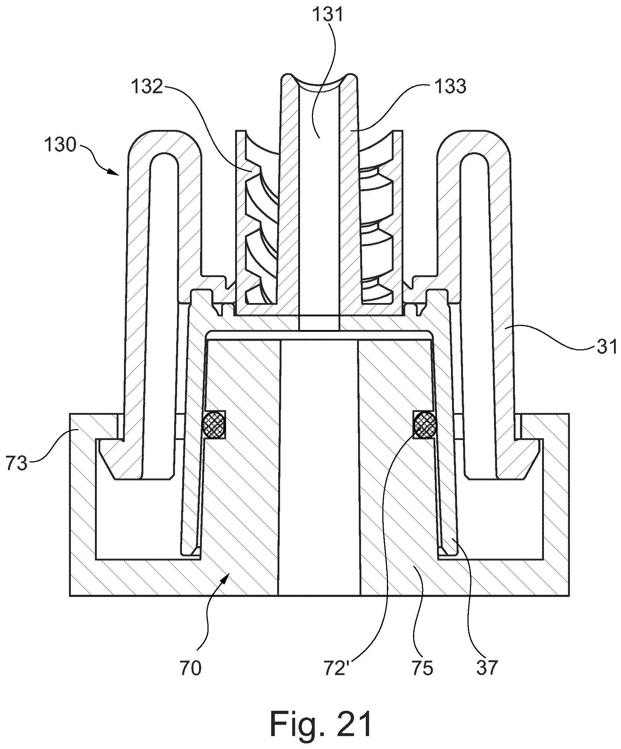

The transducer protector further comprises a second connector for fluidly coupling the second end 13 of the fluid path to a pressure transducer interface, such as one of the pressure transducer interfaces shown in Fig. 3 - 9 and 16, or to an adapter according to the first aspect as described in Fig. 18 to 20, or to an adapter according to the second aspect as shown in Fig. 21.

If not indicated otherwise, the second connector 30 comprises a second tubular element 37 for sealingly connecting to a fluid path of the pressure transducer interface. If not indicated otherwise, the second tubular element 37 has a larger diameter than the first tubular element 20 forming the first connector and/or a larger diameter than the membrane 60.

According to a first aspect of the present invention, the second connector 30 of the present invention is configured as a snap-fit connector. In the embodiment shown, the snap-fit connection between the second connector and the pressure transducer interface or the adapter according to the first aspect as described in Fig. 18 to 20 is engaged by an axial movement of the transducer protector, as indicated by arrow 40 in Fig. 2. In the context of the following discussion of the first aspect, we will always refer to the adapter according to the first aspect.

For closing the snap-fit connection, no rotating movement is needed. In contrast to the prior art solutions using a Luer connection, fixation is ensured by a single axial movement. This provides an improved usability.

The snap-fit connection is provided by snap-fit elements 31 and 32 of the second connector 30. In the embodiments, the snap-fit elements are provided as snap-fit arms of the cantilever type, wherein at least a section of the snap-fit arm extends from a connection point 34 with the main body of the transducer protector towards the pressure transducer interface or the adapter in a direction along the fluid path 11 of the transducer protector.

In the embodiments shown, the snap-fit arms 31 and 32 form or comprise handling sections for the transducer protector, which are used for gripping the transducer protector and for operating the snap-fit connection, in particular for releasing the snap-fit connection by applying pressure to the handling sections. This provides an enlarged handling area and therefore an improved usability with respect to the prior art.

Further, due to the snap-fit connection, a proper connection is ensured by tactile and audible feedback. In particular, the snap-fit connection will provide a click sound and an abrupt jump in the counter pressure against an axial movement when the snap-fit connection is engaged.

The snap-fit arms 31 and 32 are provided with a snap-fit geometry 33 at their free end, which is configured for engaging with a counter element 73, 82 or 83 or 92 or 120 of the pressure transducer interface or the adapter.

The snap-fit geometry 33 of each snap-fit arm in particular comprises a hook section 36, in particular provided by a step surface projecting away from a main outer or inner surface of the snap-fit arm, as well as a chamfer section 35, in particular in the form of a ramp, which forms the first contact with the counter element.

In particular, due to the contact of the chamfer section 35 with the counter element, the snap-fit arm will be deformed by an axial movement of the transducer protector to allow the hook section 36 to pass the counter element, in order to engage with a hook section of the counter element.

For this purpose, the chamfer section 35 is provided at the free end of the snap-fit arm in front of the hook section 36.

In one embodiment, the hook section 36 comprises a circular arc and has an annular shape. I.e., the width of the hook section is constant in radial direction.

In another embodiment of the present invention, the hook section 36 comprises a circular arc and has a crescent or sickle shape. I.e., the width of the hook section in radial direction is not constant but has its maximum at the middle of the arc.

In most of the embodiments shown, the main body of the transducer protector is provided by the two tubular sections 20 and 37 of the first and the second connector 20 and 30.

In all the embodiments shown, at least two snap-fit arms 31 and 32 are provided on opposite sides of the main body with respect to an axis provided by the fluid path.

In all the embodiments shown, the fluid path defines a single axis through the connector, with the first tubular element 20 and the second tubular element 37 being arranged co-linear with respect to each other.

Further, in all the embodiments but for the embodiment shown in Fig. 9, the snap-fit arms 31 and 32 extend from the connection point 34 with the main body along an outer side of the main body but separately from the main body and with a distance thereto towards the pressure transducer interface or the adapter. In particular, they may extend from the connection point 34 with the main body along an outer side of a tubular element forming the second end of the fluid path but separately from the tubular element and with a distance thereto.

Thereby, the length of the snap-fit arms in an axial direction can be nearly as big as the length of the main body or the fluid path, thereby increasing the handling area available on the snap-fit arms.

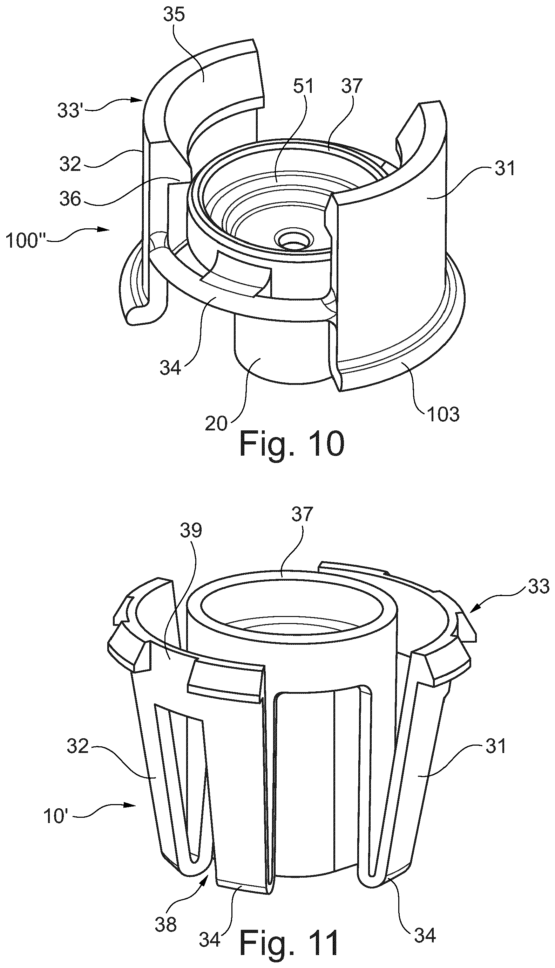

In the first embodiment shown in Fig. 1 - 5 and 7, as well as the embodiments shown in Fig. 11 to 15, the transducer protector has the snap-fit geometry 33 arranged on an outer side of the snap-fit arms 31 and 32, and therefore engages with the counter elements of the pressure transducer interface or the adapter on its outside.

For disengaging the snap-fit connection, the snap-fit arms 31 and 32 are therefore flexed with their free ends facing the pressure transducer interface or the adapter towards each other and/or towards the main body.

Further, in these embodiments, the snap-fit arms 31 and 32 only extend in one axial direction from their connection point 34 with the main body, i.e. from the connection point 34 towards the pressure transducer interface or the adapter. Further, the handling section of the snap-fit arms is provided between the connection point 34 and the free end facing the pressure transducer interface or the adapter.

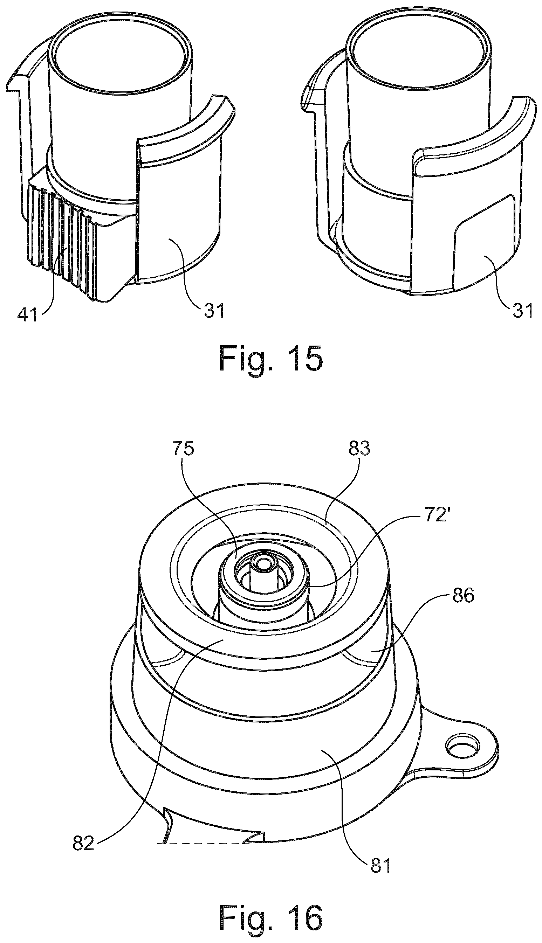

In the most of embodiments shown, the handling section is provided with ridges extending from the connection section 34 towards the free end. The ridges may alternatively be provided in a direction vertical to a direction from the connection point 34 and the free end. Further, as shown in Fig. 14 and 15, the handling section of the snap-fit arms 31 and 32 may be provided as a smooth surface without ridges.

In the embodiments shown, the snap-fit arms extend, when not deformed by external pressure, with an angle different from zero away from the central axis of the transducer protector from the connection point 34 towards their free end facing the pressure transducer interface or the adapter. In particular, the angle may be between 2 ° and 25 °, in particular between 4 ° and 15 ° as an average over the extension of the snap-fit arm. This allows an easy manufacture by injection molding.

In the embodiments shown, the second tubular section 37 is connected to the first tubular section 20 by a step portion 51. Further, the inner diameter of the second tubular section 37 is larger than the outer diameter of the first tubular section 20.

Further, In the embodiments shown, the second tubular section 37 extends from the first end of the fluid path to the step portion 51 over its entire circumference.

In the embodiments shown in Fig. 1 to 5, 11 and 12, the second tubular section 37 extends beyond the step portion 51 further towards the first side in first parts of its circumference, with the connected end of the snap-fit arms 31 and 32 attached to the rear side of these first parts of the tubular element 37. In these embodiments, the second tubular section 37 stops at the step portion in second parts of its circumference arranged between the first parts.

Further, the first parts of the second tubular element 37 are connected to the first tubular element 20 by webs 22 extending in an axial direction between the step portion 51 and the first end, and extending in a radial direction between the first tubular element 20 and the second tubular element 37.

According to a further aspect of the present invention, the main body comprising the fluid path of the first and third to sixth embodiment of the transducer protector is formed by injection molding as a single, integral part. In particular, the first and the second tubular elements may be formed by injection molding as a single, integral part.

Further, in the first and the third to sixth embodiment, all the entire body of the transducer protector including all the elements of the first and the second connector are formed by injection molding as a single, integral part.

As described in more detail in the following, in the second and seventh embodiment of the transducer protector, the main body is formed by two parts or shells connected to each other.

In the embodiments, according to another aspect of the present invention, the hydrophobic membrane 60 is bonded to a step portion 51 of the transducer protector from the second side.

In all the embodiments but the embodiment shown in Fig. 8 and Fig. 13 to 15, the diameter of the fluid path on the second side is larger than the diameter of the membrane 60 to allow for a free insertion of the membrane and accessibility for a bonding tool through the second end of the fluid path during manufacture. The bonding of the hydrophobic membrane to the sealing layer may done by heatbonding, ultrasonic welding or glueing. The preferred method for bonding the membrane to the sealing layer is ultrasonic welding.

Further, in the first embodiment as well as the fourth to seventh embodiment, the diameter of the second tubular element 37 is larger than the diameter of the membrane 60, with the membrane 60 being bonded to the step portion 51 connecting the first end of the tubular element 37 to the first tubular element 20.

Further, in all the embodiments, the step portion 51 has, at a position where the membrane is bonded thereto, a larger inner diameter than a connection hole in a bottom portion of the step portion connecting the inside of the second tubular element 37 with the inside of the first tubular element 20. In particular, the bottom of the step portion may be recessed with respect to a step surface to which the membrane is bonded, with the connection hole to the first tubular element being provided in the bottom wall.

In the embodiment, the step portion 51 also has, at a position where the membrane is bonded thereto, a larger inner diameter than the first tubular element 20.

In the seventh embodiment shown in Fig. 13 to 15, the main body of the transducer protector is formed by two parts 42 and 43. The first part 42 comprises the first end of the fluid path and in particular the first tubular element 20, as well as the snap-fit arms 31 and 32. The second part 43 comprises the second end of the fluid path and in particular the second tubular element 37.

In the seventh embodiment, the second tubular element 37 comprises a bottom section 45 extending parallel to the membrane 60 and protecting the membrane 60 from the second end of the fluid path. The bottom section 45 has a hole for allowing fluid communication between the membrane and the second end. The membrane is arranged between the step portion 51 of the first part and the bottom section 45 of the second part.

In the seventh embodiment, the membrane is bonded to the step portion 51, as described above, with the step portion forming part of the first part 42. Alternatively, the membrane could be bonded to the bottom section 45 on the side of the bottom section facing the step portion, or could be bonded between the step portion 51 and the bottom section 45.

The second tubular element 37 has, together with the bottom section 45, the shape of a pot, with second tubular element 37 extending from the outer edge of the bottom section 45 in the direction of the second end, with the diameter of the second tubular element 37 remaining equal or increasing from the bottom section towards the second end. Further, the diameter of the second tubular element 37 is, over its entire extension, larger than the diameter of the membrane 60.

In the seventh embodiment, the first part 42 and the second part 43 are connected to each other, e.g. by gluing or welding, in particular by friction or ultrasonic welding. The first and the second part can e.g. each be formed by injection molding as a single part. In the embodiment, the second part 43 has a ring shaped connection portion 44 extending towards the first part 42 and mating with a groove provided at the first part. In the embodiment, the ring shaped connection portion 44 is formed by the lower end of the tubular element 37, which protrudes beyond the position of the bottom section 45 towards the first part. The groove mating with the connection portion 44 is formed in the step portion 51 of the first part 42.

In the seventh embodiment, the second tubular element 37 extends beyond the ends of the snap-fit elements 31 and 32 in the direction of the first end, in order to provide an improved guiding of the connector when it is connected to the pressure transducer interface 70 or the adapter.

As shown in Fig. 14 and the left hand side of Fig. 15, in a first variant, the transducer protector comprises, in addition to the snap-fit arms 31 and 32 having their own handling sections for disengaging the transducer protector, second handling sections 41 arranged between the snap-fit arms 31 and 32 for gripping the transducer protector during connection with the pressure transducer interface 70 or the adapter. In the embodiment, the second handling sections 41 are arranged on the first part 42. Further, they have a radial extension that is larger than the radial extension of the snap-fit arms 31 and 32, such that they protrude to the outer side with respect to the snap-fit arms 31 and 32. In the embodiment, the outer surface of the second handling sections 41 has a concave shape, in particular in a section that is perpendicular to the axis of the transducer protector, and is provided with ridges for better gripping.

As shown on the right hand side of Fig. 15, in a second variant, the transducer protector does not comprise second handling sections 41, such that the transducer protector is also gripped at the snap-fit arms 31 and 32 for connecting it with the pressure transducer interface 70.

Apart from a transducer protector, the present invention also provides a novel pressure transducer interface configured for engaging with the inventive transducer protector. Different variants of such an interface 70, 70' and 70" are shown in Fig. 3-5 and 16. As described in more detail with respect to the adapter shown in Fig. 18 to 20, the same configuration as shown for the pressure transducer interfaces described in the following may also be used as a third connector for the adapter.

In the embodiments, the interface or the third connector is configured to engage with the transducer protector of the first or fifth and sixth embodiment. The counter element 73 of the transducer interface therefore is provided with a snap-fit geometry and in particular a hook section on its inner side, engaging with the snap-fit geometry of the transducer protector provided on the outside of the snap-fit arms of the first embodiment shown in Fig. 1 - 5 and of the fifth to seventh embodiment shown in Fig. 11 to 15.

The sealing between the transducer protector and the pressure transducer interface is provided, in all the embodiments, by a sealing element 72, 72' arranged on the side of the pressure transducer interface. The sealing element 72, 72' is configured to sealingly engage with the second tubular section 37 of the transducer protector. In particular, the sealing element 72, 72' is provided by an O-ring made from an elastomeric material.

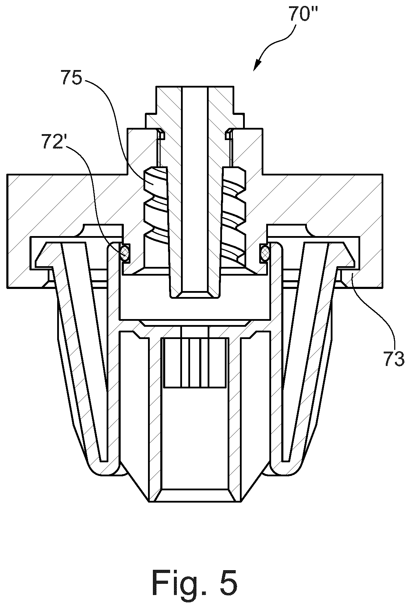

In the embodiments shown in Fig. 3 and 4, the sealing element 72 engages with a front surface of the tubular element 37. In Fig. 5 and in Fig. 13 and 16, the sealing element 72' engages with an inner side surface of the tubular element 37. In an alternative configuration, it could also engage with an outer side surface of the tubular element 37.

In Fig. 3 and 4, the sealing element 72 has a rectangular cross-section and is provided in a groove facing the transducer protector.

In Fig. 5, 13 and 16, the sealing element has a circular cross-section and/or is provided in a groove provided on the outside of a tubular section of the pressure transducer interface.

In some of the embodiments shown, the transducer protector of the present invention can be used with different kinds of machine interfaces, as shown in Fig. 3 - 5.

Fig. 3 shows a first embodiment, which is specifically designed to connect with the transducer protector of the present invention only. It comprises a projection 75 projecting into the tubular section 37 to reduce the air volume of the connector. Further, in this configuration, the membrane is better supported against accidental overpressure, because the front surface of the projection 75 is arranged close to the membrane 60 provided on the step portion 51 of the transducer protector as an abutment.

A fluid path 71 connected to the pressure transducer extends through the projection 75.

The interfaces shown in Fig. 4 and 5 and 13, 16 are configured to connect both to the transducer protector of the present invention, as well as to a prior art transducer protector comprising a standard Luer connection.

For this purpose, the interfaces shown in Fig. 4 and 5 and 13, 16 comprise a threaded section 75 and a male Luer cone comprising the fluid path 71 connected to the pressure transducer, in order to connect to a female Luer connection with a screw connection.

In the embodiments shown in Fig. 3-5, the counter element of the snap-fit connection is provided within a recess 74. Such a recess may for example be provided as a recessed portion in a main surface of the interface.

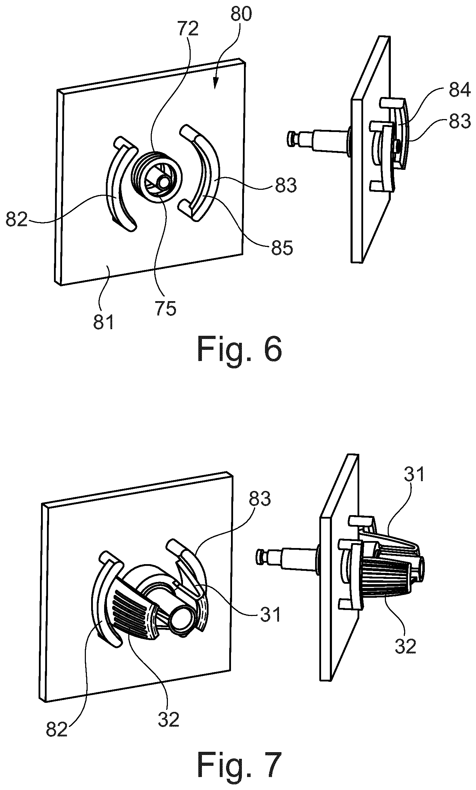

In contrast, Fig. 6 and 7 and 16 show an improved configuration of the interface that is easier to clean and disinfect, because the counter elements 82 and 83 for the snap-fit connection are provided by brackets projecting from the main surface 81 of the interface.

In particular, the brackets 82 and 83 are only connected at their ends to the main surface 81 by webs 86, and have a free bridge-like inner section that extends with a distance to the main surface 81 above the main surface 81. The hook section 84 of the counter element for engaging with a hook section 36 of the snap-fit arms 31, 32 is provided on this middle section of the brackets 82 and 84, as can be seen in Fig. 6 and 7 in the right-hand side drawing. Further, the brackets equally have a chamfer section 85 for contacting the chamfer section of the snap-fit arms of the transducer protector.

The brackets therefore can be accessed by a user from below to easily clean and disinfect around the fluid connection provided between the brackets.

In the embodiments shown in Fig. 6 and 7, the brackets 82 and 83 are separate elements which are separately connected to the main surface 81 by webs provided at their respective ends. In contrast, in the embodiment shown in Fig. 16, the brackets 82 and 83 are joined at their ends and form a ring-shaped element that is connected to the main surface by the webs 86.

As can be seen from Fig. 16, the pressure transducer interface may be provided as an interface element that can be inserted into an opening in a wall section of a medical device from behind.

The fluid connection as well as the sealing for the embodiment shown in Fig. 6 and 7 can be configured in any of the variants shown in Fig. 3 and 5. In the embodiment shown, the fluid connection is configured as shown in Fig. 5, i.e. with a threaded Luer connection 75 and an external O-ring 72. Alternatively, the fluid connection and the sealing could be configured as shown in Fig. 3 and 4, as well.

As particularly visible in Fig. 2 as well as in Fig. 6 and 7, the outer circumference of the snap-fit arms 32 and 31 is circular when seen in a cross-section in all the embodiments. Therefore, the snap-fit geometry is equally an arc section when seen in a section perpendicular to the axes of the transducer protector. This configuration provides a centering effect of the transducer protector when it is axially pressed in direction 40 towards the counter element on the machine interface side.

As shown in Fig. 6 and 7 and 16, the brackets are therefore formed by semi-circular elements or a common circular element. The recesses shown in Fig. 3 - 5 for the counter elements equally have a circular or semi-circular configuration.

Further variants of the transducer protector and the pressure transducer interface or adapter are now described with respect to Fig. 8 - 12. In the following, only those aspects in which these variants differ from the embodiments described so far are described in detail. Otherwise, the description of the embodiments provided above also refers to the embodiments shown in Fig. 8-12.

While the first embodiment of the transducer protector described so far has a snap-fit geometry on the outside of the snap-fit arms, the second to fourth embodiment shown in Fig. 8 - 10 comprises the snap-fit geometry on the inner side of the snap-fit arms and therefore engages with a counter snap-fit geometry arranged on an outer side of the counter element.

In particular, the snap-fit geometry 33' comprising the chamfer section 35 and the hook section 36 is provided on an inner side of the snap-fit arms 31 and 32, for engaging with a hook section 92 provided on the outside of a tubular element of the pressure transducer interface or adapter.

Further, the snap-fit arms 31 and 32 have a first section extending from the connection point 34 with the main body towards the first side of the transducer protector 100, 100' and 102', and a second section extending from the connection point 34 towards the second end and the pressure transducer interface or adapter. Thereby, by applying a pressure on the outer side of the first section, the first section of the snap-fit arms can be flexed to an inner side towards the main body, leading to movement of the second section comprising the snap-fit geometries 33' away from the main body, releasing the snap-fit connection. Therefore, in the embodiments shown in Fig. 8 - 10, the handling section 103 is provided on the first section between the connection 34 with the main body and the free end of the first section facing the first end. In particular, the handling section 103 is provided by a ring section projecting from the free end of the first section towards the outside.

The fourth embodiment shown in Fig. 10 is otherwise similar to the first embodiment shown in Fig. 1 - 5, with the main body being formed by the first tubular element 20 of the first connector and the second tubular element 37 of the second connector, the step section 51 arranged therebetween to which the membrane is bonded from the second side. The bonding of the hydrophobic membrane is done by heat bonding, ultrasonic welding or gluing. The preferred method for bonding the membrane to the sealing layer is ultrasonic welding.

In this embodiment, the two snap-fit arms 31 and 32 extend along the outer surface of the tubular section 37 with a gap being formed between the outer surface of the tubular section 37 and the inner surface of the snap-fit arms 31 and 32 over their entire axial length, in order to simplify the mold for injection molding the transducer protector. The two snap-fit arms 31 and 32 are connected to each other via a connection element extending between the two snap-fit arms and connected to the tubular section 37 at a position between the two snap-fit arms. In the embodiment, the connection element is ring-like.

The sealing and the fluid connection of this transducer protector can be configured as shown in Fig. 3-7, and only requires a different configuration of the counter element for the snap-fit geometry with a hook section provided on an outside rather than an inside of the counter element.

Both in the first and the fourth embodiment, as well as the fifth and the sixth embodiments, which will be described later on, the entire transducer protector is injection molded as an integral element, with the membrane being provided from the second side of the fluid path.

In contrast, in the embodiment shown in Fig. 8, the main body is made by two shells similar to the prior art solution, with the membrane 60 arranged between two shells at an enlarged middle section arranged between the first tubular section 20 of the first connector and the second tubular section 37 of the second connector, the two shells being welded to each other. In this embodiment, the snap-fit arms 31 and 32 are attached with their connection points 34 on the outer circumference of the enlarged middle section 102.

Further, the fluid connection between the second tubular section 37 and the interface is not provided by an O-ring, as in the other embodiments, but by a Luer connection between the tubular section 37 and the Luer cone 76 of the standard Luer connection, which is also provided with a standard screw section 75 for engaging with a standard Luer connector.

Therefore, the interface shown in Fig. 8 is compatible both with a prior art Luer connection of a transducer protector as well as the second embodiment of the inventive transducer protector using a snap-fit connection.

In contrast, in the embodiment shown in Fig. 9, the interface 90' is configured to be used only with an embodiment of a transducer protector according to the present invention.

In this embodiment also, an external snap-fit element is used, as already described with respect to Fig. 8 - 10. In this embodiment, the sealing is provided by an elastomeric component 65 which is part of the transducer protector, with the membrane being over-molded on the elastomeric component. In particular, the pressure transducer interface may comprise a conical section 91 engaging with a conical section provided at the second end of the fluid path, with the elastomeric material being provided as an elastomeric layer on the cone section of the transducer protector. In this embodiment, the connection 34 between the snap-fit arms is provided on an outer circumference of the cone section of the transducer protector, with the cone section being connected to the tubular section 20 by a step portion. The elastomeric material may in particular be a TPE. Therefore, in this embodiment, the membrane is bonded to the main body of the pressure transducer from the second side essentially at the second end of the fluid path.

In the embodiments shown in Fig. 8 and 9, the snap fit arms 31, 32 are connected to the main body of the transducer protector at a circumferential position of the main body between the snap fit arms 31, 32, as in the embodiment shown in Fig. 10.

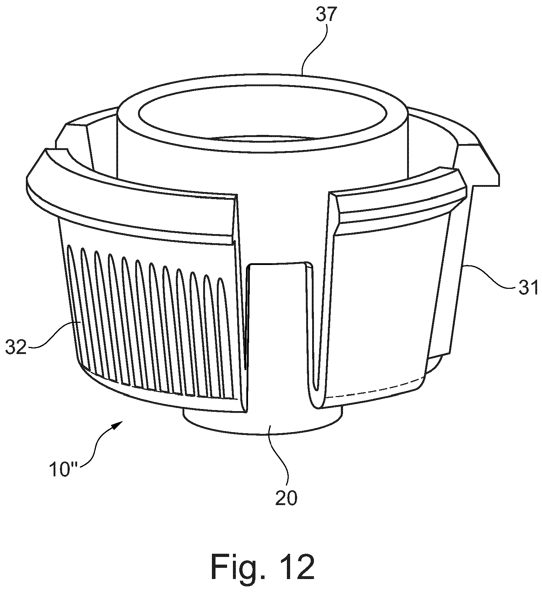

Fig. 11 and 12 show two variants of the first embodiment of the transducer protector. Both embodiments have an internal snap-fit element as the first embodiment, and the same general configuration.

The embodiment shown in Fig. 11 has a reduced mass and material consumption than the first embodiment, with cutouts 38 provided in the snap-fit arms 31 and 32 extending from the first side towards the second side, thereby splitting the circumference of the snap-fit arms into several arm sections on the first side thereof. In the area of the cutouts 38, the second tubular section 37 equally ends already at the step section 51 and does not further extend towards the first side.

The distal ends of the remaining sections of the snap-fit arms are connected at the second side of the transducer protector by a partial ring section. The snap-fit geometry is provided, in the embodiment, only where the material of the snap-fit arms extends, while it is not provided in the circumferential portions where there are the cutouts 38.

In the embodiment shown in Fig. 12, two additional wings or snap-fit arms 39 are provided in between the snap-fit arms 31 and 32. The user however only has to press the snap-fit arm 32 and 32' to remove the transducer projector, and the additional wings 39 are included only to improve the alignment if the counter element of the pressure transducer interface or adapter is provided as a circular recess.

In the embodiment, a snap geometry is provided on the free end of the wings 39 for snapping into the counter element. However, the snap angle of the snap geometry of the wings 39 is not 90 degrees, such that they will disengage just due to the axial removal movement, and do not need to be pressed for disengaging the snap connection.

In contrast, the snap connection provided by the snap arms 31 is only disengaged, in all the embodiments, by applying a pressure on the snap-fit arms in a radial direction. In particular, the snap angle of the snap geometry 33 and in particular of the hook section 36 as well as the corresponding hook section on the counter element is, for the first and the second snap fit arm 31 and 32, essentially at 90 degrees.

In all the embodiments, due to the snap-fit connection, the present invention provides a decisively easier handling. Further, for those embodiments where the main body of the transducer protector is provided as a single piece to be manufactured by injection molding, or the membrane is bonded to the main body from the second side of the fluid path, the manufacture is simplified with respect to the two-shell-design of the prior art solution.

Fig. 17 shows a tubing set 200 comprising a transducer protector 10 connected by line 220 to a component of an extracorporeal blood tubing set. In the embodiment, the component to which the transducer protector 10 is connected by line 220 is a venous air trap chamber 210. Connected to chamber 210 are connections lines 230 and 240 for connection to a venous line and an arterial line, as well as further connection lines 250.

The end of line 220 is inserted and glued into connector 20 of the transducer protector 10. The distal ends of connection lines 230, 240 and 250 are provided with connection elements for providing a releasable connection to further lines or components of the extracorporeal blood circuit, such as Luer-lock connectors.

Fig. 18 to 20 show an embodiment of a system and an adapter 110 according to the first aspect of the present invention.

The system comprises a transducer protector according to the first aspect described above, i.e. having a second connector configured as a snap-fit connector.

In the embodiment shown in Fig. 18, the transducer protector corresponds to the seventh embodiment shown in Fig. 13 to 15. We therefore refer to the description of the transducer protector according to the first aspect and in particular to the description of the seventh embodiment above. However, we note that all other embodiments of the transducer protector according to the first aspect may also be as the transducer protector of the system according to the first aspect.

In the system according to the first aspect, the transducer protector is not directly connected to a pressure transducer interface, such as a pressure transducer interface of a blood treatment device, but to an adapter 110 which provides the connection to the pressure transducer interface 150.

Therefore, the snap-fit counter elements as described above are provided on the adapter.

Specifically, the adapter 110 comprises a second main body comprising a second fluid path 111, a third connector for releasably coupling a first end of the second fluid path 111 to the first fluid path of the transducer protector, and a fourth connector for releasably coupling a second end of the second fluid path 111 to the pressure transducer interface 150. In the embodiment, the second fluid path 111 extends axially through the adapter.

According to the first aspect, the third connector is counter-element for the snap-fit connector of the transducer protector.

Therefore, all the configurations of the pressure transducer interfaces described with respect to Fig. 3 -9, 13 and 16 may be provided on the third connector of the adapter rather than on the pressure transducer interface.

In the embodiment, the first end of the second fluid path 111 of the adapter 110 is configured as a third tubular element 112 to be sealingly engaged with the second end of the first fluid path of the transducer protector.

In the embodiment, the counter elements 120 of the transducer interface are provided with a snap-fit counter geometry and in particular a hook section.

In the embodiment shown in Fig. 18 to 20, the third connector is configured to connect to a transducer protector of the first embodiment shown in Fig. 1 - 5 and of the fifth to seventh embodiment shown in Fig. 11 to 15, having a snap-fit geometry provided on the outer side.

In the embodiment, the snap-fit counter geometry and in particular the hook section is therefore provided on the inner side of the counter elements 120, engaging with the snap-fit geometry 33 of the transducer protector provided on the outside of the snap-fit arms 31 of the first embodiment shown in Fig. 1 - 5 and of the fifth to seventh embodiment shown in Fig. 11 to 15.

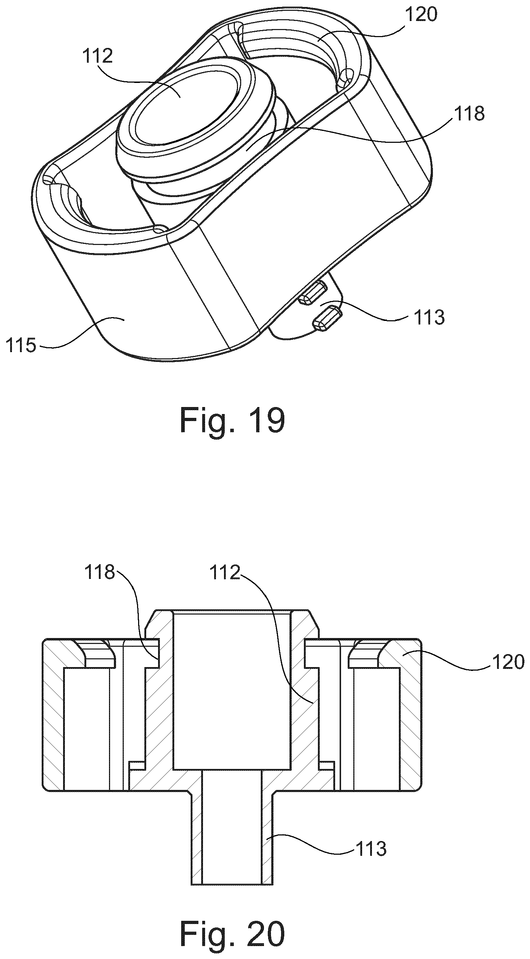

Further, the snap-fit counter geometry is provided with a chamfer on its entry side, see Fig. 19 and 20, engaging with the chamfer of the snap-fit element 31 when the transducer protector is inserted into the adapter.

The hook section and the chamfer section extend circumferentially in an arc section around an axis defined by the second fluid path 111 at its first end.

The sealing between the transducer protector and the adapter 110 is provided by a sealing element 72' arranged on the side of the adapter.

The sealing element 72' is configured to sealingly engage with the second tubular section 37 of the transducer protector. In particular, the sealing element 72' is provided by an O-ring made from an elastomeric material.

In the embodiments shown in Fig. 19, the sealing element 72' engages with an inner side surface of the tubular element 37. In an alternative configuration, it could also engage with an outer side surface of the tubular element 37 or with a front surface of the tubular element 37.

In Fig. 18, the sealing element 72' is provided in a groove 118 provided on an outer surface of the third tubular element 112.

In the embodiment, the third tubular element 112 has an outer surface to engage with an inner surface of the second tubular element 37, with the outer surface being cone-shaped. The tubular element as well as the cone shape will help to guide the second tubular element on the third tubular element in order to avoid a misalingement.

In the embodiment, in an axial direction, a counter-geometry of the counter-elements 120 is provided between a first axial end and a second axial end of the outer surface of the third tubular element 112.

In a radial direction, the counter-element 120 is provided on an outer side of the third tubular element 112 with a free space provided between the counter-element 120 and the third tubular element 112 to accommodate the second tubular element 37 and the a snap-fit connection elements 31 of the second connector.

As shown in Fig. 18 to 20, the third connector comprises two counter-elements 120 to engage with corresponding connection elements of the second connector, the counter elements 120 being arranged on opposite sides of an axis defined by the second fluid path 111 at its first end. The snap-fit connection provided by the third connector is therefore releasable by pressing the handling sections of the snap-fit elements 31 from an outer side in a direction towards each other.

The adapter 110 comprises an outer wall 115, with the counter-elements 120 are arranged on a free end of the outer wall 115 facing inwards. The outer wall 115 extends at a distance to the third tubular element 111 and is connected to the third tubular element 112 by a bottom wall 117, such that the outer wall 115 an the bottom wall 117 together have a cup shape with the third tubular element 112 extending from the bottom wall towards the open side of the cup shape.

In an embodiment of the present invention, the wall and/or the bottom wall are formed as closed surfaces. Alternatively, the wall and/or the bottom wall may have openings and/or may be provided by web elements.

In particular, the counter elements may be configured as brackets, such as shown in Fig. 6 and 7 and 16. The configuration shown in this embodiment may be used identically for the adapter, with the counter elements 120 for the snap-fit connection being provided by brackets projecting from the bottom wall 117 in exactly the same way as they project from the main surface 81 of the interface shown in Fig. 6, 7 and 16.

In particular, the brackets of the adapter may only connected at their ends to the bottom wall 117 by webs, and have a free bridge-like inner section that extends with a distance to the bottom wall 117 above the bottom wall 117. The hook section of the counter element for engaging with a hook section 36 of the snap-fit arms 31, 32 is provided on this middle section of the brackets. Further, the brackets may equally have a chamfer section for contacting the chamfer section of the snap-fit arms of the transducer protector.

In the embodiment shown in Fig. 18, fourth connector of the adapter is configured to sealingly engage the pressure transducer interface 150. The pressure transducer interface 150 is a different connector type than the pressure transducer interfaces according to the first aspect described so far. Therefore, the fourth connector is of a different connector type than the third connector.

The pressure transducer interface 150 and the fourth connector may be any type of connector, and in particular may be a standard connector as used in the art. The adapter 110 thereby allows to engage the transducer protector having a snap-fit connector to a pressure transducer interface 150 having a connector type to which the snap-fit connector of the transducer protector could not be connected.

In an embodiment of the present invention, pressure transducer interface 150 and the fourth connector are Luer type connectors. In particular, the fourth connector is configured as a female Luer connector and the pressure transducer interface 150 is configured as a male Luer connector.

In the embodiment, the fourth connector has a sealing cone, which is in particular provided on the inside of the fourth tubular element 113 forming the second end of the second fluid path 111, as well and a screw element 116 which in particular may be provided on the outer surface of the fourth tubular element 113 to engage with corresponding geometries of the pressure transducer interface 150.

The pressure transducer interface 150 comprises a male cone 151, and a tubular element 152 arranged to surround the male cone 151 and having screw threads provided on its inner side.

In the embodiment, the second fluid path 111 extends along a single direction through the adapter 110. Further, the second fluid path 111 is not provided with a membrane, but leads through the adapter without any barrier.

In the embodiment, the adapter is integrally formed as a single element, in particular by injection molding.

The pressure transducer interface 150 is arranged an outer surface 160 of a blood treatment machine 170, and in particular integrated in the casing of the blood treatment machine. The blood treatment machine is in particular a dialysis machine.

The pressure transducer 160 is arranged inside the blood treatment machine 170 and fluidly connected to the pressure transducer interface 150.

Fig. 21 shows an embodiment of an adapter 130 according to the second aspect of the present invention.

The adapter 130 equally comprises a second main body comprising a second fluid path 131, a third connector for releasably coupling a first end of the second fluid path 131 to the first fluid path of a transducer protector, and a fourth connector for releasably coupling a second end of the second fluid path 131 to the pressure transducer interface. In the embodiment, the second fluid path 131 extends axially through the adapter.

According to the second aspect, the adapter 130 is configured to snap-fit connect to a pressure transducer interface having a snap-fit counter element, such as described above with respect to Fig. 3-9, 13 and 16. Therefore, the fourth connector of the adapter 130 may be configured in exactly the same way as the second connector of the transducer protector of the first aspect.

In the embodiment shown in Fig. 21, the fourth connector of the adapter 130 is configured as the second connector of the transducer protectors according to the first embodiment shown in Fig. 1 - 5 and the fifth to seventh embodiment shown in Fig. 11 to 15. We therefore refer to the above description with respect to the configuration of the fourth connector.

In particular, the fourth connector comprises connection elements 31 connected to a main body of the adapter and having a free end comprising a connection geometry for snap engaging the counter element 73 of the pressure transducer interface. The connection elements are snap-fit arms than can be flexed to provide and disconnect the snap-fit connection.

The second end of the second fluid path 131 is configured as a fourth tubular element 37 to be sealingly engaged with a fluid path of the pressure transducer interface 70. The connection elements 31 of the fourth connector extend from a connection point with the main body of the adapter towards the pressure transducer interface 70 on an outside and with a distance to the fourth tubular element 37.

The connection geometry of the at least one connection element 31 is arranged on an outer side of the connection element with respect to a radial direction and comprises a chamfer section and a hook section. The snap-fit geometry is therefore configured to snap engage a counter element of the pressure transducer interface from an inner side.

In the embodiment, the fourth connector comprises two separate snap-fit elements arranged on opposite sides of an axis defined by the second fluid path at its second end, such that the snap-fit connection is releasable by pressing handling sections of the snap-fit elements 31 from an outer side in a direction towards each other.

In the embodiment, a handling section of the snap-fit elements 31 of the adapter are arranged between a connection point where the snap-fit element is connected to the main body of the adapter and a free end of the snap-fit element configured for connecting to the pressure transducer interface.

The fourth connector of the adapter may however also have any one of the alternative configurations of the second connector of the transducer protectors of the first aspect described above.

In contrast to the transducer protectors of the first aspect described above, the third connector of the adapter according to the second aspect is configured to releasably engage with a second connector of a transducer protector having a different type of connector.

In particular, according to the second aspect, the third connector of the adapter allows to connect a standard transducer protector as known from the prior art, in particular one having a Luer type connector. In particular, I may allow to connect a transducer protector such as shown in Document EP 2 226 087 A1, the content of which is included herein by reference in its entirety, which therefore may form the transducer protector of the system according to the second aspect.

Therefore, according to the second aspect, the second an third connectors may be matching Luer type connectors. In particular, the third connector may be configured as a male Luer cone connector and the second connector as a female Luer connector.

As shown in Fig. 21, the third connector has a sealing cone 133 and a screw element 132 to engage with corresponding geometries of the second connector. In particular, the third connector comprises a male sealing cone 133 forming the first end of the second fluid path 131 and a third tubular element extending around the male sealing cone 133 and comprising a screw element 132 arranged on its inner surface.

In the embodiment, the second fluid path 131 extends along a single direction through the adapter.

In the embodiment, the adapter does not comprise a membrane and the second fluid path 131 leads through the adapter without a barrier.

The adapter may be integrally formed as a single element, in particular by injection molding. Alternatively, the adapter may be formed from two parts connected with each other, as described for the transducer protectors of the first aspect.

The adapter of the second aspect may be configured in exactly the same way as the transducer protectors of the first aspect, but with the third connector described above and without the membrane.

## Claims

1. A system for connecting a pipe of an extracorporeal circuit to a pressure transducer interface, the system comprising a transducer protector and an adapter, the transducer protector comprising:
- a first main body comprising a first fluid path;
- a first connector for fluidly coupling a first end of the first fluid path to the pipe of the extracorporeal circuit;
- a second connector for releasably coupling a second end of the first fluid path to the adapter; and
- a hydrophobic gas-permeable membrane arranged in the first fluid path for separating the first end of the first fluid path from the second end;
the adapter comprising:
- a second main body comprising a second fluid path;
- a third connector for releasably coupling a first end of the second fluid path to the first fluid path of the transducer protector;
- a fourth connector for releasably coupling a second end of the second fluid path to the pressure transducer interface,
wherein the second connector is a snap-fit connector and the third connector comprises a snap-fit counter-element for the second connector.

2. The system according to claim 1, wherein the first end of the second fluid path of the adapter is configured as a third tubular element to be sealingly engaged with the second end of the first fluid path of the transducer protector,
wherein preferably, the adapter comprises a sealing element to sealingly engage with the second end of the first fluid path of the transducer protector and in particular with a second tubular element of the transducer protector forming the second end of the first fluid path of the transducer protector,
wherein preferably, the sealing element is provided on an outer surface of the third tubular element to engage with the inner surface of a second tubular element of the second connector,
wherein preferably, the sealing element is a sealing ring arranged in a groove provided on the outer surface of the third tubular element.

3. The system according to claim 2,
wherein the third tubular element has an outer surface to engage with an inner surface of the second tubular element, wherein preferably, the outer surface is cone-shaped, and/or wherein preferably, in an axial direction, a counter-geometry of the counter-element is provided between a first axial end and a second axial end of the outer surface of the third tubular element,
and/or
wherein in a radial direction, the counter-element is provided on an outer side of the third tubular element with a free space provided between the counter-element and the third tubular element to accommodate the second tubular element and/or a snap-fit connection element of the second connector.

4. The system according to any one of the preceding claims, wherein the counter-element has at least one snap-fit counter-geometry to snap-fit engage with a connection element of the second connector,
wherein the snap-fit counter-geometry is arranged on an inner side of the counter-element to engage with a snap-fit geometry arranged on an outer side of the connecting element,
and/or
wherein the snap-fit counter-geometry comprises a hook section for snap engaging with a hook section of the snap-fit geometry of the second connector and preferably a chamfer section, wherein preferably, the hook section and/or the chamfer section extend circumferentially in an arc section around an axis defined by the second fluid path at its first end.

5. The system according to any one of the preceding claims, wherein the third connector comprises at least two counter-elements to engage with corresponding connection elements of the second connector,
wherein preferably, the counter-elements are arranged on opposite sides of an axis defined by the second fluid path at its first end,
and/or
wherein preferably, the at least two counter-elements are arranged on a wall extending circumferentially around the first end of the second fluid path, wherein preferably, the wall extends at a distance to a third tubular element of the third connector, the third tubular element forming the second fluid path at its first end, wherein preferably, the at least two counter-elements extend from a free end of the wall to an inside towards the second fluid path.

6. The system according to any one of the preceding claims, wherein the fourth connector is configured to sealingly engage a pressure transducer interface,
wherein preferably, the fourth connector is of a different connector type than the third connector, wherein preferably the fourth connector is a Luer connector and/or has a sealing cone and a screw element to engage with corresponding geometries of the pressure transducer interface, wherein preferably, the fourth connector is configured as a female Luer connector,
and/or
wherein preferably, the fourth connector comprises a fourth tubular element forming the second end of the second fluid path, wherein preferably, the fourth tubular element has an inner surface configured as a sealing cone and a screw element arranged on its outer surface.

7. A system for connecting a pipe of an extracorporeal circuit to a pressure transducer interface, the system comprising a transducer protector and an adapter, the transducer protector comprising:
- a first main body comprising a first fluid path;
- a first connector for fluidly coupling a first end of the first fluid path to the pipe of the extracorporeal circuit;
- a second connector for releasably coupling a second end of the first fluid path to the adapter; and
- a hydrophobic gas-permeable membrane arranged in the first fluid path for separating the first end of the first fluid path from the second end;
the adapter comprising:
- a second main body comprising a second fluid path;
- a third connector for releasably coupling a first end of the second fluid path to the first fluid path of the transducer protector;
- a fourth connector for releasably coupling a second end of the second fluid path to the pressure transducer interface,
wherein the fourth connector is a snap-fit connector configured to be connected to a counter-element of the pressure transducer interface.

8. The system according to claim 7, wherein the fourth connector comprises at least one connection element connected to a main body of the adapter and having a free end comprising a connection geometry for snap engaging the counter element of the pressure transducer interface,
wherein preferably, the connection element extends from a connection point where it is connected to the main body of the adapter in a direction substantially along of the second fluid path of the adapter towards the pressure transducer interface,
and/or
wherein preferably the connection geometry of the at least one connection element of the fourth connector is arranged on an outer side of the connection element with respect to a radial direction,
and/or
wherein preferably the connection geometry comprises a chamfer section and a hook section,
and/or
wherein preferably the fourth connector comprises at least one snap-fit element of the cantilever type, wherein the snap-fit element preferably is a snap-fit arm connected to a main body of the adapter and having a free end comprising a snap-fit geometry for snap engaging the counter element of the pressure transducer interface, wherein further preferably, the snap-fit arm extends from a connection point where it is connected to the main body of the adapter in a direction substantially along of the second fluid path of the adapter.

9. The system according to claim 7 or 8, wherein the second end of the second fluid path is configured as a fourth tubular element to be sealingly engaged with a fluid path of the pressure transducer interface,
wherein preferably the at least one connection element of the fourth connector extends from a connection point with the main body of the adapter towards the pressure transducer interface on an outside and further preferably with a distance to the fourth tubular element,
and/or

10. The system according to any one of claims 7 to 9,
wherein the fourth connector comprises at least two separate connection elements, the connection elements being preferably arranged on opposite sides of an axis defined by the second fluid path at its second end,
and/or
wherein the snap-fit connection provided by the fourth connector is a releasable connection, wherein preferably, the fourth connector is configured such that the snap-fit connection is releasable by flexing at least one snap-fit element by applying pressure to a handling section of the snap-fit element, wherein the snap-fit element is preferably configured to be releasable by a pressure acting on the handling section of the snap-fit element from an outer side in a direction towards the main body of the connector,
and/or
wherein the fourth connector comprises at least two separate snap-fit elements, the snap-fit elements being preferably arranged on opposite sides of an axis defined by the second fluid path at its second end, wherein further preferably, the snap-fit connection provided by the second connector is releasable by pressing the handling sections of the snap-fit elements from an outer side in a direction towards each other,
and/or
wherein a snap-fit geometry of the fourth connector is configured to snap engage a counter element of the pressure transducer interface from an inner side, wherein preferably, a handling section of at least one snap-fit element of the adapter is arranged between a connection point where the snap-fit element is connected to the main body of the adapter and a free end of the snap-fit element configured for connecting to the pressure transducer interface.

11. The system according to any one of claims 7 to 10, wherein the third connector is configured to sealingly engage with the second connector of the transducer protector, wherein preferably, the third connector is of a different connector type than the fourth connector, wherein the third connector preferably has a sealing cone and a screw element to engage with corresponding geometries of the second connector, wherein preferably, the third connector is configured as Luer connector, in particular as a male Luer cone connector,
and/or
wherein the third connector comprises a male sealing cone forming the first end of the second fluid path and a third tubular element extending around the male sealing cone and comprising a screw element arranged on its inner surface.

12. The adapter of a system according to any one of the preceding claims.

13. An extracorporeal blood tubing set comprising a pipe and a system according to any one of claims 1 to 11, with the pipe being connected to the first connector, wherein preferably the pipe is permanently connected to the first connector.

14. A blood treatment machine, in particular a dialysis machine, comprising a pressure transducer, a pressure transducer interface and an adapter according to claim 12 and/or a system according to any one of claims 1 to 11, wherein the adapter and the pressure transducer interface are configured to be releasably connected to each other.

15. Use of system according to any one of claims 1 to 11 and/or an adapter according to claim 12 for connecting a pipe of an extracorporeal circuit to a pressure transducer,
wherein preferably, the transducer protector is discarded after each use or a plurality of uses and the adapter remains arranged on the pressure transducer interface to be used consecutively with a plurality of transducer protectors,
or wherein the adapter is discarded together with the transducer protector after each use or a plurality of uses.
